# EUROPEAN PATENT APPLICATION

(11) **EP 2 141 621 A2**
(43) Date of publication of application: **06.01.2010**
(21) Application number: 09164215.7
(22) Date of filing: 30.06.2009
(51) Int. Cl.: G06F 19/00

(54) **Automatically pre-populated templated clinical daily progress notes**

(30) Priority: 30.06.2008 US 77161 P
(71) Applicant: The Regents of The University of California, Oakland, California 94607-5200 (US)
(72) Inventor: Martin, Neil A., Encino, CA 91436 (US); Buxey, Farzad D., Marina del Rey, CA 90292 (US); Vesselin, Zlatev, Aliso Viejo, CA 92656 (US); Xiao, Hu, Los Angeles, CA 90034 (US)
(74) Representative: Witte, Weller & Partner

(57) **Abstract**

A system and method for providing a pre-populated daily progress note to a health care provider in response to a request for such a note or on a predetermined schedule. The system includes a server, and databases in communication with the server. The server is connected to a communication network. Software on server the generates a pre-populated daily progress note for a patient by retrieving clinical data related to that patient and populating the corresponding fields of the template. The system transmits the daily progress note to a health care provider who may enter additional data into the template. The health care provider transmits the completed daily progress note to the hospital server.

## Description

### CROSS-REFERENCE TO RELATED APPLICATIONS

The present application claims the benefit under 35 U.S.C. §119(e) of the Provisional Patent Application Serial No. 61/077,161 filed June 30, 2008 which is herein incorporated by reference.

### FIELD OF THE INVENTION

The invention relates to accessing clinical data and its transfer to and from a health care provider within a clinical setting. In particular, the invention involves delivery of clinical data after its completion into an automatically pre-populated templated daily progress note.

### BACKGROUND OF THE INVENTION

Documentation is a critical step in any medical care setting. Health care providers which may include referring physicians, nurses, medical residents, the medical records department, insurance companies, hospital administrators and staff, among others, all use portions of clinical data including patient demographics, laboratory results, as well as vital signs. Health care providers gather clinical data during rounding visits with patients in a comprehensive daily progress note. The daily progress note is the building block for quality patient care and monitoring. Clinical data, and the daily progress note into which the clinical data is incorporated, enables the health care provider to evaluate a patient's condition over a period of time, and to determine treatment strategies such as prescribing new medications, changing existing medications, and providing other therapeutic interventions.

A daily progress note may take either a paper or electronic form. There are many different versions of daily progress notes. A typical note is traditionally one page in length, and includes blank spaces, or fields, for the health care provider to complete, i.e. fill in, based on observations and analyses made while observing a patient during a rounding visit. In addition, the note typically includes fields for demographic data related to the patient, for example, hospital number, hospital location, admitting diagnosis, number of days in the hospital, number of days post-operative, and titles and dates of previous surgery.

The specific layout and types of information included on a daily progress notes vary based on a number of factors, for example the condition for which the patient has sought treatment, the hospital, the health care provider, and so forth. A daily progress note may include fields for specific laboratory results which have been determined to be significantly relevant to treatment of a specific medical condition.

While visiting each patient during daily rounds, a health care provider makes substantive and objective observations of the patient and records them in the fields of the daily progress note. The health care provider typically uses one daily progress note for each individual patient. The daily progress note for each patient will become a permanent record in the hospital. A health care provider can use a group of daily progress notes to track a patient's condition over a set period of time, for example a week. The daily progress note may be used by the billing department to render a bill for services provided by the health care provider, and by professionals to monitor the condition and treatment of a patient.

A disadvantage of known systems and methods is that in order to gather and access clinical data to include in the daily progress note health care providers must locate and access a hospital installed computer. This is especially true when the health care provider must include demographic data, laboratory results, and other types of clinical data not typically available to the health care provider prior to observation of the patient during a daily round. Accessing this data at a hospital computer terminal is time consuming and inconvenient, especially when the health care provider is at the patient's bedside or other point of care.

Another disadvantage of known systems and methods is that clinical data is generally entered manually by a health care provider composing a daily progress note. Entering data manually, however, can slow down follow-up treatment. In addition, manual data entry increases the portion of the health care providers work day dedicated to ministerial tasks.

Another disadvantage of manual data entry, whether in a paper note or electronic note, is that it is prone to errors in transcription. For example, the health care provider may incorrectly transcribe data.

There is a desire for a system and method for providing pre-populated daily progress note to a health care provider by email at a specified time or in response to a request by the health care provider.

### SUMMARY OF THE INVENTION

It is accordingly an object of the present invention to provide a system and method that overcomes the limitations of the prior art.

It is a further object of the present invention to provide a system and method for providing a pre-populated daily progress note to a health care provider.

It is a further object of the present invention to provide a system and method for providing a pre-populated daily progress note to a health care provider on a push delivery basis.

It is a further object of the present invention to provide a system and method for providing a pre-populated daily progress note to a health care provider on an on demand (i.e. pull) basis.

It is a further object of the present invention to provide a system and method for generating a pre-populated daily progress note for a specific patient by merging clinical data associated with the patient and stored on a hospital network database with a daily progress note template.

It is a further object of the present invention to provide a system and method automatically transmitting to a health care provider a plurality of pre-populated daily progress notes, wherein each of the plurality of pre-populated daily progress notes includes clinical data associated with a patient for which the health care provider is responsible.

It is a further object of the present invention to provide a system and method for providing pre-populated daily progress notes to a health care provider at a specific time. For example, it is an objection of the present invention to provide pre-populated daily progress notes to a physician on a daily basis at a set time before the physician's daily rounds.

It is yet a further object of the present invention to provide a system and method for providing pre-populated daily progress note to a health care provider in response to a specific request.

It is yet a further object of the present invention to provide a system and method for generating a patient specific pre-populated daily progress note based on a specific condition of the patient.

It is yet a further object of the present invention to provide a system and method for transmitting a pre-populated daily progress note to a health care provider via email, IMS, or SMS.

It is yet a further object of the present invention to provide a system and method for transmitting a pre-populated daily progress note to a health care provider, wherein the pre-populated daily progress note is encrypted to secure transmittal.

It is yet a further object of the present invention to provide a system and method for receiving a completed daily progress note from a health care provider, and storing the completed daily progress note in a "sign and store" application. Such an application provides the health care provider with an opportunity to review and revise the completed daily progress note after the health care provider has finished his daily rounds, and before submitting a final signed copy of the completed daily progress note.

It is yet a further object of the present invention to provide a system and method for receiving a completed daily progress note and transmitting the daily progress note, or portions thereof, to one or more third parties. For example, the system may transmit a portion of the note to a billing department, a portion to a referring physician, and the entire daily progress note to a data repository in communication with the hospital network.

It is yet a further object of the present invention to provide a system and method for updating a clinical database with clinical data components included in a completed daily progress note.

These and other objects of the present invention are achieved by a system and method for automatically providing pre-populated daily progress notes to a health care provider. In some embodiments, the system includes a server, a clinical data database in communication with the server, and a template database in communication with the server, the template database having at least one template. The templates include one or more fields corresponding to clinical data components related to a condition of a patient. The system further includes software executing on the sever for merging clinical data components related with a patient into the template, and further includes software for transmitting the merged template to the health care provider on either a push delivery basis or an on-demand delivery basis.

In some embodiments, the system includes software executing on the server for transmitting a merged template to a health care provider via electronic mail, IMS, or SMS. In yet further embodiments the merged template comprises a plurality of clinical data components associated with a first patient. In yet other embodiments the transmitted email comprises a plurality of merged templates, and each of the merged templates includes clinical data associated with a different patient's care, for which a health care provider is responsible. In yet other embodiments of the present invention each of the merged templates are associated with a plurality of patients assigned to a health care provider. In some embodiments of the present invention the software transmits the merged templates to the health care provider on pre-determined time each day before the health care provider is scheduled to begin daily rounds.

These and other objects and advantages of the present invention will become more apparent from the following detailed description considered with reference to the accompanying drawings.

### BRIEF DESCRIPTION OF THE DRAWINGS

FIG. 1 illustrates a system and method for automatically providing pre-populated daily progress notes to a health care provider according to one embodiment of the present invention.

FIG. 2 illustrates a system and method for automatically providing pre-populated daily progress notes to a health care provider according to one embodiment of the present invention, and a reviewing and signing application for revising daily progress notes completed by the health care provider.

FIG. 3 illustrates a sample of a template of a daily progress note according to one embodiment of the present invention.

FIG. 4 illustrates another sample of a template of a daily progress note according to one embodiment of the present invention.

FIG. 5 illustrates a method for automatically providing a pre-populated clinical daily note to a health care provider.

FIG. 6 illustrates a method for receiving a pre-populated template for a daily progress note, and entering data into the pre-populated daily progress note.

FIG. 7 illustrates a method of authenticating a completed daily progress note according to one embodiment of the present invention.

FIG. 8 illustrates a method of reviewing, editing, and authorizing a completed daily progress note according to one embodiment of the present invention.

### DETAILED DESCRIPTION OF THE INVENTION

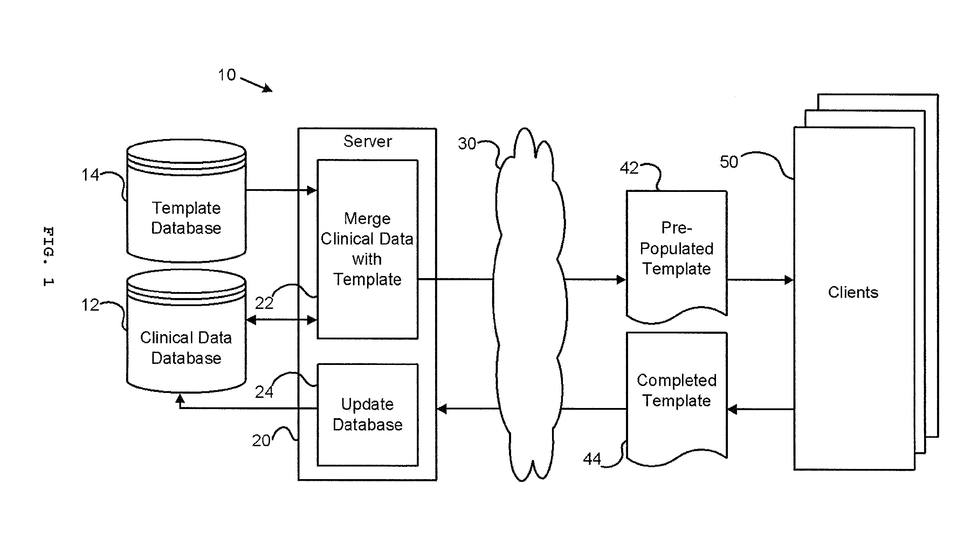

FIG. 1 illustrates a system 10 for automatically providing pre-populated daily progress notes to a health care provider over a network communication according to one embodiment of the present invention. The system 10 includes a server 20. The server 20 is in communication with a network 30. In some embodiments the server 20 may be a personal computer. In other embodiments the server 20 may be a plurality of servers in communication. For example, the server 20 may be part of a hospital data network.

The system illustrated in FIG. 1 includes at least one clinical data database 12 in communication with the server 20. The clinical data database 12 includes a plurality of clinical data components. Clinical data, and its constituent components, includes any data related to the treatment and care of a patient, for example data related to the condition of a patient, billing and payment history related to a patient, and a medical history data of the patient. For the purposes of this application clinical data also refers to severity scores, and data trends calculated to monitor and evaluate one or more patients. Clinical data is collected through a variety of systems and methods and stored in the clinical data database 12. The clinical data database 12 provides the system 10 with clinical data for a number of patients.

In some embodiments, clinical data components comprise information relating to the condition of a patient. In yet further embodiments of the present invention each clinical data component includes a patient identifier, wherein a patient is associated with the identified component of clinical data. For example, the patient identifier may include a number, code, name, or like tag. It should be understood that many different clinical data structures may be used with the present invention.

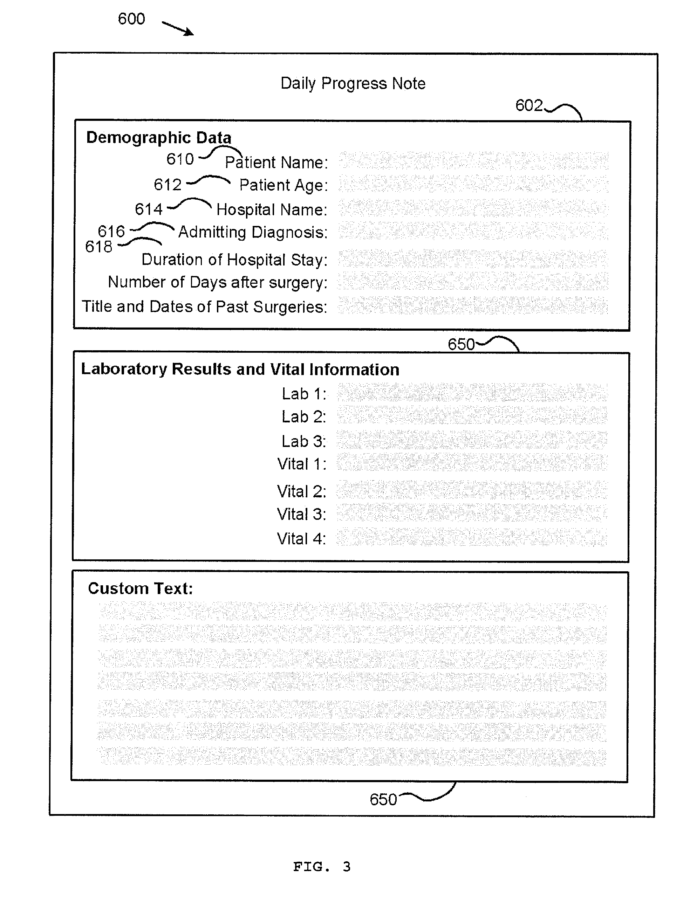

In further reference to FIG. 1, the system 10 includes at least one template database 14 in communication with the server 20. The template database 14 includes at least one template 500, 600. In the embodiment shown in FIG. 1, the template is a daily progress note. In reference to FIG. 3, a template for a daily progress note 600 in accordance with one embodiment of the present invention is shown. The template 600 includes one or more fields, e.g. 611, 613, 615, 617, corresponding to one or more clinical data components, e.g., 610, 612, 614, 616. In the embodiment shown in FIG. 3, the fields are separated into different sections 602, 650, 670 based on a category of the clinical data. For example, the template 600 includes a demographic data section 602. This section 602 includes fields corresponding to demographic clinical data. For example patient name 610, patient age 612, hospital name 614, admitting diagnosis 616, and duration of hospital stay 618.

In further reference to FIG. 3, the template 600 includes additional sections 650, 670. For example the template 600 includes a clinical data section corresponding to laboratory results and vital information 650. The daily progress note template 600 also includes a section 670 having one or more fields for custom text 556, 562. In some embodiments of the present invention software executing on the server 20 automatically pre-populates the fields of the template 600 with clinical data components prior to transmitting the template 600 to a client 50. The pre-populated template 42 is transmitted to the client 50 via a communication network 30. The pre-populated template 42 is displayed on an interface of the client 50. In some embodiments the client 50 includes an interface for user input and output of data, for example a touchscreen.

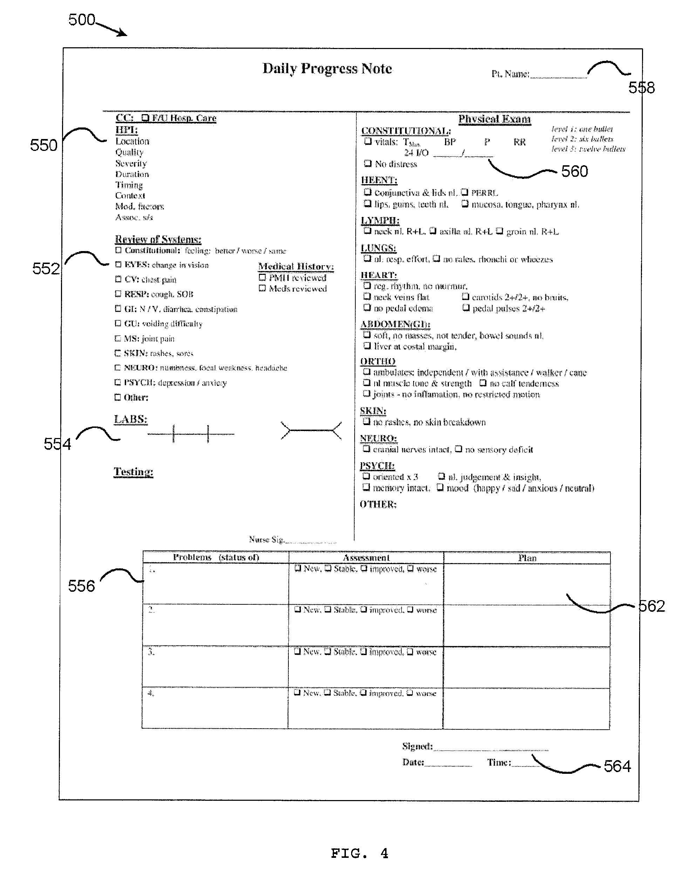

In reference to FIG. 4 another embodiment of a template of a daily progress note 500 is illustrated. The template 500 includes a plurality of fields, e.g., 550, 558, 560, corresponding to different components of clinical data. For example, the template 500 includes a field for patient name 558, vitals 560, status of health problems 556, plan for treating health problem 562, labs 554, review of systems, for example eyes 552, and location of patient 550. In addition, the template 500 includes a field for the health care provider's signature 564. It should be understood that there are many different variations of daily progress notes. It should further be understood that many different data fields are possible. The templates 500, 600 shown in FIGS. 3-4 are for the purpose of illustrating a daily progress note template.

In reference to FIG. 1, the template database 14 is in communication with the server 20. The template database 14 includes at least one template. For example, the template database 14 may include a single standardized daily progress note template for a particular health care facility. In other embodiments the template database 14 includes a plurality of templates, each having a different form. For example, the template database 14 may include a subset of one or more daily progress notes templates having clinical data fields relating to a specific type of patient, or a specific type of ailment the patient is suffering. In other embodiments the template database 14 may include a subset of templates having clinical data fields related to a specific department of a health care facility.

In further reference to FIG. 1, the server 20 is connected to a communication network 30. For example, in some embodiments the server 20 is in communication with a local network of a health care facility. In other embodiments the server 20 is in communication with the Internet. In further reference to FIG. 1 the system 10 includes one or more clients 50 in communication with the network 30. The client 50 may include, but is not limited to, a desktop computer 152, a notebook computer 164, a tablet computer 154, a personal digital assistant 162, and a mobile phone 166. A client 50 in some embodiments is a device that primarily handles input (e.g., user interaction) and output (e.g., displaying), while processing is done on a separate server. In other embodiments processing of the clinical data is done on the client 50.

In further reference to the system 10 shown in FIG. 1, the system 10 comprises software executing on the server 20 for merging clinical data components with a daily progress note template, thereby automatically forming a pre-populated daily progress note template 42. The pre-populated daily progress note template 42 includes at least some clinical data related to the patient, while other fields of the template 42 are blank. The system 10 further includes software executing on the server 20 for transmitting the pre-populated template 42 to a client 50 via the communication network 30. In some embodiments the software transmits the pre-populated template 42 to the client 50 via an email. In other embodiments the software transmits the pre-populated template 42 to the client 50 via text message ("SMS"), or an instant message ("IMS"). In some embodiments of the present invention the client retrieves the pre-populated template using by accessing a remote web server.

In preferred embodiments software executing on the server 20 generates a pre-populated template 42 for a first patient. Software executing on the server 20 queries the template database 14 for a template corresponding to the first patient. The software executing on the server 20 queries the clinical data database 12 for clinical data components corresponding to one or more fields of the template retrieved by the template query. The software 20 forms a pre-populated template 42 by merging clinical data components into corresponding fields of the template.

In some embodiments the system generates a plurality of pre-populated templates 42 in accordance with the requirements of a first health care provider, for example a first physician. The plurality of pre-populated templates 42 correspond to a group of patients for which the first physician is responsible for providing care. Thus, the system 10 provides the first physician daily pre-populated progress notes 42 for each patient on the physician's rounding list.

In some embodiments the system 10 transmits the pre-populated template 42 to the client 50 via the communication network 30 on a push delivery basis. For example, in some embodiments the system 10 pushes the pre-populated template 42 to the client 50 thirty minutes prior to the health care provider's daily rounding shift. In other embodiments the pre-populated templates 42 are generated and transmitted to a rounding physician on a push basis, such as to provide the physician with the most up to date clinical information as the physician observes, diagnosis, and treats a patient. For example, the system 10 transmits the pre-populated templates 42 to the physician on a push basis as the physician progress through his daily rounds. It should be understood that many different push schedules are possible with the present invention.

In some embodiments the system 10 transmits the pre-populated template 42 to the client 50 via the communication network 30 on an on demand basis. For example, in some embodiments a health care provider, requests a pre-populated template 42 via the client 50. In response the system 10 transmits the requested pre-populated template 42 to the client 50. In this way the health care provider can request an updated pre-populated daily progress note template 42 as he visits each patient on his daily rounding list, thereby providing the most up to date clinical data in the daily rounding note.

The pre-populated template 42 is displayed on an interface of the client 50. For example, the template 42 is displayed on a touchscreen tablet. The health care provider can enter additional clinical data components to the pre-populated template 42 using the interface, for example, a keyboard, touch screen, or other input device on the client 50. In reference to the daily progress note template 500 shown in FIG. 4, the rounding physician can enter data related to the patient, for example, the status of problems with the patient 556 and a plan for treating the identified problem 562. In some embodiments the client 50 includes a keyboard or voice control for entering clinical data components into the daily progress note 42. In other embodiments, the template includes one or more fields with drop down menus, thereby providing a series of clinical data components for the health care provider to select.

In further reference to FIG. 1, after the health care provider is finished with a round and the health care provider has entered clinical data into the pre-populated template 42, the template serves as record of the daily round. The client 50 transmits the completed template 44 to the server 20 via the communication network 30. Software executing on the server 20 receives the completed template 44 from the client 50. The client 50 can use any known means to transmit the completed template 44 to the network. For example, the client can transmit the completed template via email, SMS, IMS. In some embodiments the client can access the server 20 through a remote web application and transmit the completed template 44 to the server 20.

In reference to the embodiment shown in FIG. 1, the system 10 comprises software executing on the server 20 for updating the clinical database 12 with the clinical data components added by the health care provider during the daily round. Software executing on the server 20 extracts the clinical data components and stores them in accordance with the structure of the database 12. The system 10 further comprises software executing on the server 20 for storing one or more completed templates 44 in a database in communication with the server 20. In this way, the system 10 maintains a record of a patient's daily progress through completed templates 44, and associated clinical data components.

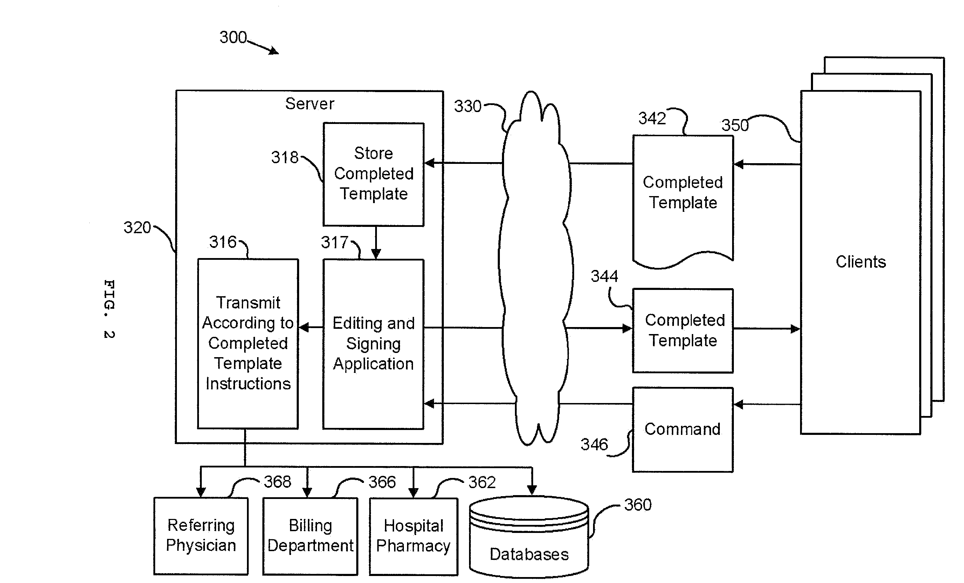

In reference to FIG. 2, another embodiment of the inventive system and method is illustrated wherein the server includes an editing and signing application 317 for revising and authorizing a completed template 342. In this embodiment the client 350 transmits a completed template 342 to the server 320 via the communication network 430. Software executing on the server 320 receives the completed template 342 and stores the completed template 342. After the health care provider completes the daily rounds the health care provider can access the completed template 342 stored on the server 320. For example, the health care provider can access completed template 342 via a remote web application or through a client in network communication with the server. In other embodiments, server 320 transmits one or more completed templates 344 to the server 350. Using the client 50, the health care provide can make revisions to completed daily progress note template 344 and verify the accuracy of the information provided therein. The health care provider authorizes the completed template 344 by transmitting a command 346 from the client 350 to the server 320. Software executing on the server 320 receives the command 346. The command 346 may be an authorization that the daily progress list is complete, and that the system 10 may disseminate the clinical data included in the daily progress note to one or more sources.

In reference to the daily progress note template 500 shown in FIG. 4, the template 500 includes a field for the health care provider's signature. Using the editing and signing application 317 the health care provider can electronically sign the daily progress note. In some embodiments the health care provider can include instructions on the template, or the original template may include certain instructions for the delivery of a completed and authorized template. For example, in reference the system 10 shown in FIG. 2, software executing on the server 320 transmits the completed template 344 to one or more sources. For example, software on the server 320 transmits the completed template to a referring physician 368, a billing department 366, a clinical data database 360, and a hospital pharmacy 362.

In some embodiments of the present invention the pre-populated template 42 is pre-configured to interface with a Computerized Provider Order Entry (CPOE). Through such a configuration a rounding physician can order medication and tests through the client for a patient as the physician completes her rounds. In this way the system 10 reduces delay and prevents errors in transcription. In yet further embodiments the pre-populated template 42 includes at least one link for displaying information related to a specific condition. For example, the pre-populated daily progress note may indicate in one or more fields that the patient has recently had a surgery. In some embodiments, this field also includes a link that provides additional clinical data related to the surgery, for example relevant images of the affected area, status regarding the surgery, and in general more detailed information than is available on the daily progress note.

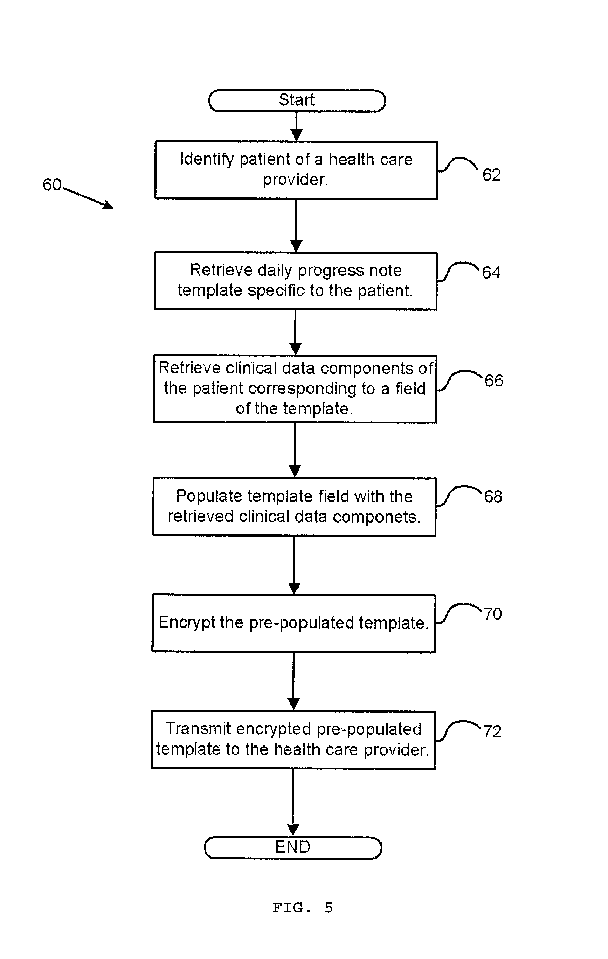

FIG. 5 illustrates a method for automatically providing a pre-populated clinical daily note client to a health care provider. This method allows for pre-scheduled pre-populated daily progress notes to be delivered to an electronic client of a health care provider on a pre-determined schedule. First, the method includes the step of identifying a patient of a health care provider 62. This information is typically included in a clinical data database, or similar database, maintained and in communication with the hospital network. The next step is to retrieve a daily progress note template specific to a patient 64. It should be understood that in the some health care facilities only a single daily progress note template exists, while in other facilities a large number of different templates exist. Next the method includes the steps of retrieving clinical data of the patient corresponding one or more fields of the selected daily progress note template 66. The method next includes the step of populate the corresponding fields with the retrieved clinical data 68. The method next includes the step of encrypting the pre-populated template 70. This step ensures that the daily progress note, and the clinical information contained therein, is secure. Finally, the method includes the step of transmitting the encrypted pre-populated template to the health care provider 72.

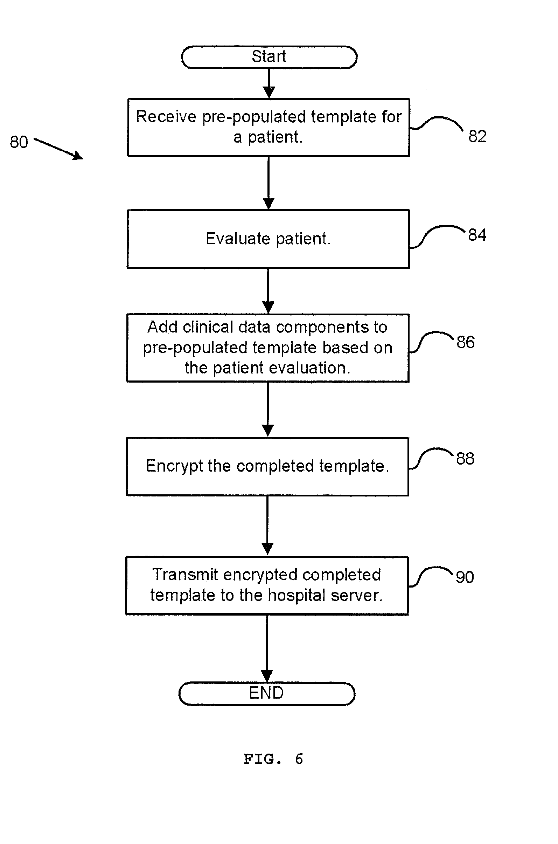

FIG. 6 illustrates a method for receiving a pre-populated template for a daily progress note, and entering data into the pre-populated daily progress note. The method first includes the step of a client receiving a pre-populated daily progress note associated with a patient 82. Next, the method includes the step of evaluating the patient 84. After evaluation, the client updates the pre-populated template by using an interface on the client to add clinical data components to the template based on the patient evaluation 86. After which, the client 50 encrypts the completed template 88. Finally the client transmits the encrypted template to a hospital server.

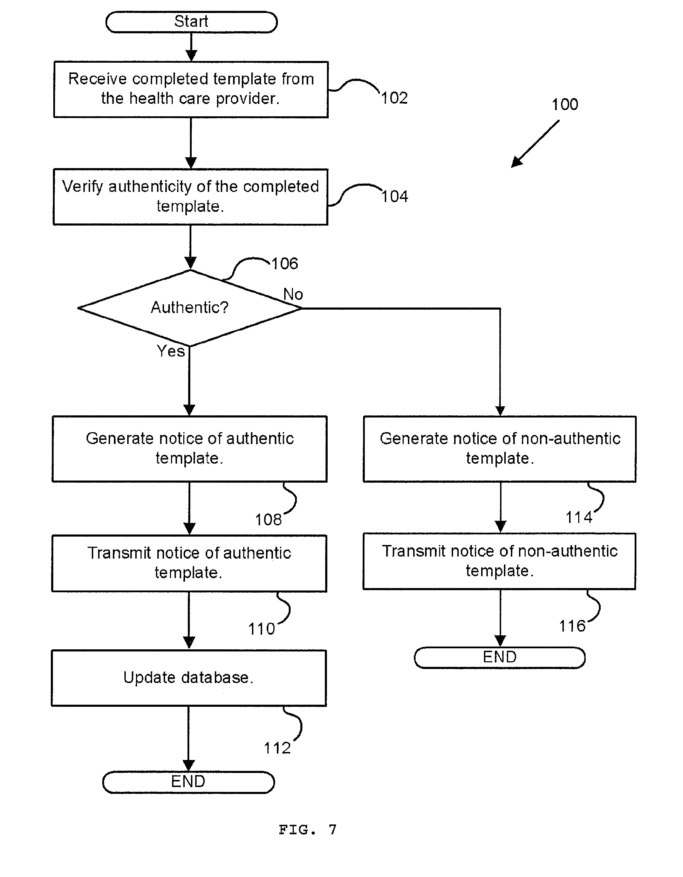

FIG. 7 illustrates a method of authenticating a completed daily progress note according to one embodiment of the present invention. As the server receives completed daily progress notes it is important to authenticate the author of the notes, as well as to make certain revisions to the note are correct before it becomes a permanent part of the record. In reference to FIG. 7 the server 20 receives a completed template from a health care provider via the client 102. The server verifies the authenticity of the completed template. In some embodiments this step occurs when the system receives direct authorization from the health care provider. If the system determines that the template is authentic, the system generates a notice of authentic template 108, transmits the notice of authentic template 110, and updates the clinical data database 112. If the system determines that the template is not authentic, the system generates a notice of non-authentic template 114, and transmits the notice of non-authentic template to the relevant health care provider.

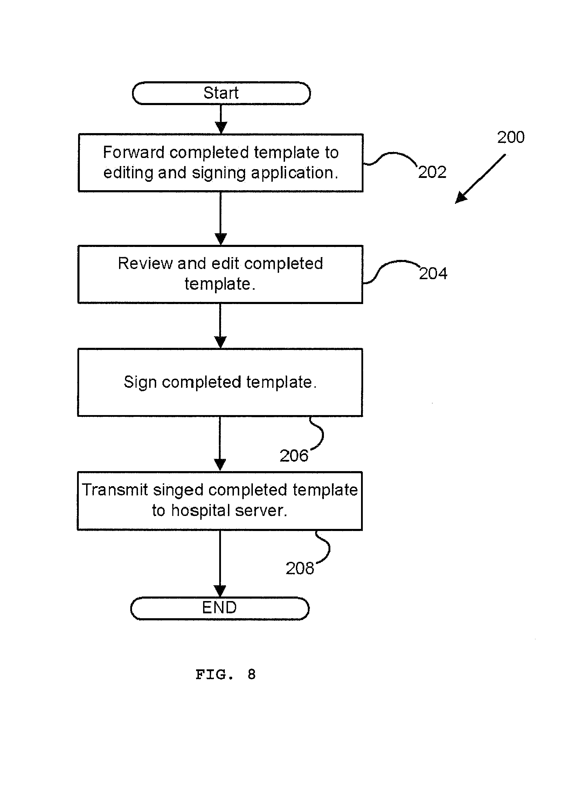

FIG. 8 illustrates a method of reviewing, editing, and authorizing a completed daily progress note 200 according to one embodiment of the present invention. The method includes the steps of forwarding a completed template to the editing and signing application 202. Reviewing and editing the completed template 204. Authorizing the completed template 206, and finally transmitting the signed completed template to a hospital server.

Although the invention has been described with reference to a particular arrangement of parts, features and the like, these are not intended to exhaust all possible arrangements or features, and indeed many modifications and variations will be ascertainable to those of skill in the art.

## Claims

1. A system for providing pre-populated daily progress notes to a health care provider, comprising:
a server;
a clinical data database in communication with said server, said database comprising a plurality clinical data components;
a template database in communication with said server, said template database comprising at least one template, said template comprising one or more fields corresponding to one or more clinical data components;
software executing on said sever for merging said one or more clinical data components with said template to form a pre-populated template;
software executing on said server for automatically transmitting said pre-populated template to a health care provider via push delivery.

2. The system for claim 1, wherein said software transmits said pre-populated template via email.

3. The system of claim 2, wherein said pre-populated template comprises a plurality of clinical data components,
wherein each said clinical data component is related to a first patient.

4. The system of claim 3, wherein said email further comprises:
a plurality of pre-populated templates, each said template comprising clinical data components related to one of a plurality of patients.

5. The system of claim 4, wherein said health care provider is responsible for providing care to said plurality of patients.

6. The system of claim 5, wherein the system automatically transmits said plurality of pre-populated templates to said health care provider on a periodic basis corresponding to a rounding schedule of said health care provider.

7. The system of claim 3, further comprising:
software executing on said server for receiving a completed template from said health care provider;
wherein said completed template comprises a plurality of clinical data components entered by said health care provider.

8. The system of claim 7, further comprising:
software executing on said server for updating said clinical data database with said plurality of clinical data components entered by said health care provider.

9. The system of claim 8, further comprising:
software executing on said server for storing said completed template;
wherein said health care provider can review said completed template stored on said server via a client in communication with said server via said network,
wherein said health care provider can authorize said completed template stored on said server.

10. The system of claim 8, further comprising:
software executing on said server for transmitting at least a portion of said completed template to one or more third parties.

11. The system of claim 10, wherein said completed template is pre-configured with an address of said one or more third parties.

12. The system of claim 5, further comprising
software executing on said server for encrypting said pre-populated template,
wherein said software encrypts said pre-populated template prior to transmitting said pre-populated template to said health care provider.

13. The system of 7, further comprising:
software executing on said server for determining an author of said entered clinical data components.

14. The system of claim 13, further comprising:
software executing on said server for generating a confirmation of receipt in response to a receipt of said completed template;
software executing on said server for transmitting said confirmation of receipt to said health care provider.

15. A system for providing pre-populated daily progress notes to a health care provider, comprising:
a server;
a clinical data database in communication with said server, said database comprising a plurality clinical data components;
a template database in communication with said server, said template database comprising at least one template, said template comprising one or more fields corresponding to one or more clinical data components;
software executing on said sever for merging one or more clinical data components with said one or more fields of said template to form a pre-populated template;
a communication link between said server and a network;
a client device;
a communication link between said client device and said network;
software executing on said server for transmitting said pre-populated template to said client device via said network.

16. The system of claim 15, further comprising
software executing on said server for receiving a request for said pre-populated template from said client device;
wherein said server transmits said pre-populated template to said client device in response to said request.

17. The system of claim 16, wherein said server transmits a plurality of pre-populated templates to said client device.

18. The system of claim 15 further comprising:
software executing on said client device for displaying said pre-populate template;
software executing on said client device for entering clinical data components into one or more fields of said pre-populated template.

19. The system of claim 15, further comprising
software executing on said server for transmitting said pre-populated template to said electronic device on a push basis.

20. The system of claim 19, wherein said software executing on said server transmits said pre-populated template to said electronic device in accordance with a daily schedule of said health care provider.

21. A method for generating and transmitting a pre-populated daily progress note, comprising the steps of:
providing an electronic daily progress note template having one or more fields;
generating a pre-populated daily progress note by merging clinical data components with said one or more fields of said daily progress note template;
transmitting said pre-populated daily progress note to a health care provider via email.

22. The method for claim 21, further comprising the step of:
transmitting said pre-populated daily progress note to said health care provider on a daily basis prior to a scheduled round of said health care provider.

23. The method of claim 22, wherein said first merged template comprises a plurality of clinical data components, each said plurality of clinical data components being associated with a first patient.
